# FASCICULE DE BREVET EUROPEEN

(11) **EP 0 639 358 B1**
(45) Date de publication et mention de la délivrance du brevet: **13.01.1999**
(21) Numéro de dépôt: 93440065.6
(22) Date de dépôt: 20.08.1993
(51) Int. Cl.: A61F 2/38

(54) **Prothèse de genou à articulation sphérique**
Knieprothese mit Kugelgelenk
Knee prosthesis with ball joint

(43) Date de publication de la demande: 22.02.1995
(73) Titulaire: SOCIETE CIVILE ESSOR, 74550 Perrignier (FR)
(72) Inventeur: Bosredon, Jean, 33000 Bordeaux (FR)
(74) Mandataire: Polus, Camille

(56) Documents cités:
- EP-A- 0 126 978
- DE-A- 4 102 509
- GB-A- 2 088 724
- US-A- 3 868 730
- US-A- 4 085 466
- US-A- 4 224 697

## Description

La présente invention a pour objet une nouvelle prothèse de l'articulation du genou, notamment, mais non exclusivement, implantable en remplacement d'une prothèse implantée antérieurement.

On connaît déjà de nombreux types de prothèses de l'articulation du genou, qu'il s'agisse de prothèses dites à glissement ou de prothèses monoblocs à axe d'articulation.

Les prothèses dites à glissement, qui sont de loin les plus confortables, comportent un implant fémoral et un implant tibial sur lequel roule et glisse ledit implant fémoral, et sont de conception très proche de l'articulation naturelle.

Ces prothèses à glissement présentent néanmoins un inconvénient, celui d'un possible déboîtement se traduisant par un tiroir antérieur et surtout latéral, cet inconvénient apparaissant notamment lorsqu'il s'agit d'une prothèse de reprise implantée en remplacement d'une prothèse antérieure.

D'autre part ces prothèses à glissement laissent apparaître dans le temps une dégradation de la partie tibiale due à l'usure du plateau généralement réalisé en polyéthylène, par frottement sur l'implant fémoral. Le préambule de la revendication 1 se réfère à une prothèse de l'art antérieur selon US-A- 3 868 730.

L'inconvénient majeur des prothèses à axe vient du manque de liberté de mouvement d'une pièce par rapport à l'autre, limitant l'articulation à ne fonctionner que dans un plan, le plan sagittal.

La présente invention vise à remédier à ces divers inconvénients en proposant une prothèse de genou à articulation sphérique, empêchant tout mouvement de translation d'une pièce par rapport à l'autre tout en autorisant leur rotation.

La prothèse de genou selon la présente invention comporte un implant tibial et un implant fémoral, et présente les caractéristiques selon la revendication 1.

Conformément à l'invention, la cupule de l'implant fémoral peut être libre de mouvement dans la cavité dudit implant fémoral, ou fixée mécaniquement par des moyens connus en soi.

Toujours conformément à l'invention, le plateau en matière synthétique de l'implant tibial peut se présenter en une seule partie ou en deux parties placées de part et d'autre de l'ergot central.

Le plateau tibial de la prothèse selon l'invention peut être solidarisé à l'embase de manière amovible par tout moyen approprié, par exemple par clippage, mais il peut aussi être simplement posé sur l'embase tibiale de manière à pouvoir se déplacer en translation ou en rotation lors des mouvements du genou, sa mobilité étant en ce cas limitée par des dispositifs de type connu en soi, par exemple des pions solidaires de l'embase.

La tête sphérique de l'implant tibial de la prothèse selon l'invention peut être solidarisée à l'ergot saillant du plateau tibial par tout moyen approprié connu en soi, par exemple par emmanchement de type conique.

L'embase tibiale de la prothèse selon l'invention peut être fixée à l'os par tout moyen approprié connu en soi, par exemple à l'aide de vis ou par encastrement, mais elle peut également comporter à sa face inférieure une tige d'ancrage intramédullaire.

La longueur de la tige intramédullaire fémorale et, le cas échéant, de la tige intramédullaire tibiale peut être variable, et elle peut être modifiée par fixation de segments de tiges à l'aide de moyens mécaniques connus en soi.

Après implantation des implants fémoral et tibial, ceux-ci sont assemblés l'un à l'autre par introduction de la tête sphérique de l'implant tibial dans la cupule hémisphérique de l'implant fémoral, ce dernier pouvant comporter une partie trochléenne prolongeant les condyles et susceptible de s'articuler avec un implant rotulien de type connu.

Pour pallier les inconvénients de laxité liés aux problèmes de coupe osseuse et de tension ligamentaire, la profondeur de la tête sphérique de l'implant tibial peut être variable, une épaisseur de plateau correspondant à chaque profondeur de tête. En fonction de la profondeur de tête choisie on peut plus ou moins éloigner le fémur du tibia pour retendre les ligaments latéraux lorsqu'ils ne sont pas lésés et retrouver ainsi la balance ligamentaire.

La cupule hémisphérique de l'implant fémoral peut être soit simplement hémisphérique, soit hémisphérique avec des bords la prolongeant au-delà du diamètre, de manière à prévenir des luxations, soit hémisphérique à rétention de façon à retenir la tête sphérique, le recouvrement de la tête sphérique devant être suffisant pour empêcher toute luxation antérieure ou postérieure d'une pièce par rapport à l'autre.

De cette manière le jeu ménagé entre la partie bicondylienne de l'implant fémoral et le plateau ou les demiplateaux de l'implant tibial n'assure aucune fonction mécanique autre que celle de limiter les mouvements dans un plan frontal (varus - valgus).

Avec le temps l'usure de la cupule, par frottement de la tête sphérique, rapproche les pièces fémorale et tibiale l'une de l'autre, et le jeu se réduit. Dès que ce jeu a disparu les condyles fémoraux viennent au contact du plateau tibial et à ce moment, les frottements ne sont plus ceux de la tête sur la cupule mais des condyles sur le plateau tibial. Ce relais permet d'augmenter la longévité de la prothèse et d'éviter au patient une nouvelle intervention chirurgicale pour changer les pièces usées.

Le tiroir antérieur et la laxité latérale impossibles et les mouvements en varus, valgus étant limités, toutes les autres rotations sous contraintes ou sans contraintes sont permises par la prothèse selon l'invention.

Les avantages et les caractéristiques de la présente invention ressortiront plus clairement de la description qui suit et qui se rapporte au dessin annexé, lequel en représente quelques modes de réalisation non limitatifs.

Dans le dessin annexé :
- la figure 1 représente une vue en perspective d'une prothèse de l'articulation du genou selon l'invention, en position de semi-flexion.
- la figure 2 représente une vue partielle de profil d'une prothèse selon l'invention dans la même position, avec coupe selon un plan médian de l'implant fémoral.
- la figure 3 représente une vue en plan de l'implant tibial de la même prothèse.
- la figure 4 représente une vue partielle de face de le même prothèse dans la même position de semi-flexion.

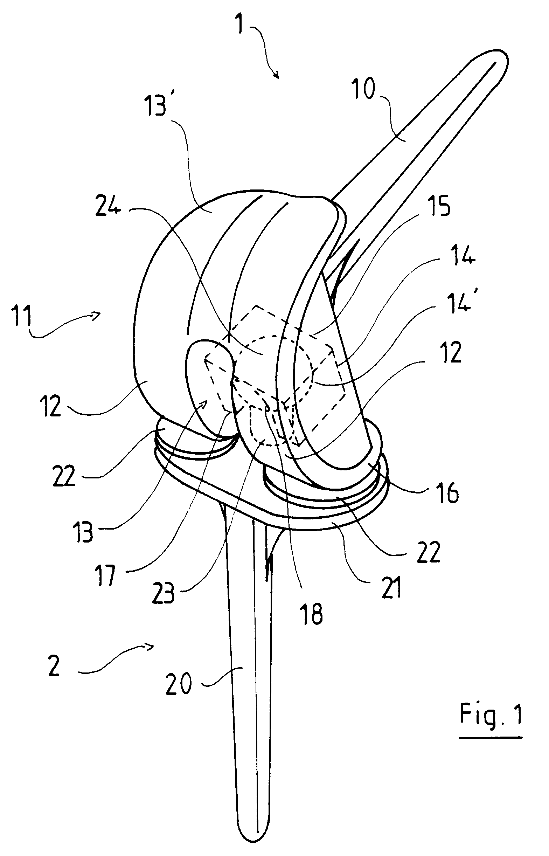

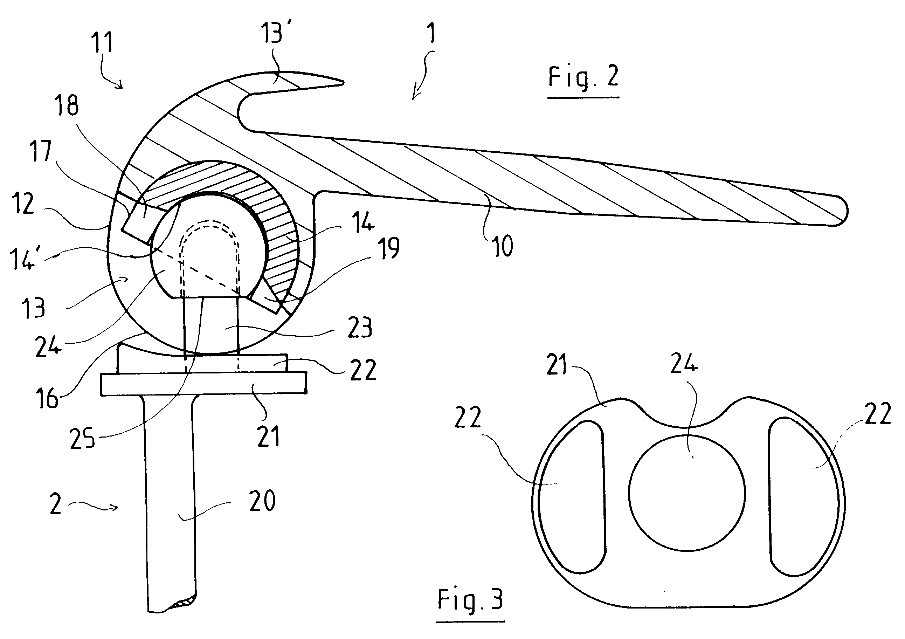

Si on se réfère aux figures 1 et 2 on peut voir qu'une prothèse du genou selon l'invention comporte un implant fémoral 1 et un implant tibial 2, ainsi bien entendu qu'un implant rotulien de type connu, non représenté.

L'implant fémoral 1 comporte une tige intramédullaire 10 terminée en zone distale par une partie bicondylienne 11 comprenant deux condyles 12 de part et d'autre d'une échancrure intercondylienne 13.

L'implant fémoral 1 peut ne pas comporter de trochlée 13', auquel cas la rotule s'articule avec la trochlée fémorale non prothésée.

Intérieurement, centralement et postérieurement la partie bicondylienne 11 comporte une cavité 15 dont l'ouverture est disposée en regard de l'échancrure intercondylienne 13 et orientée vers les condyles postérieurs 16, et dans laquelle est disposée une cupule 14 présentant une ouverture hémisphérique 14'.

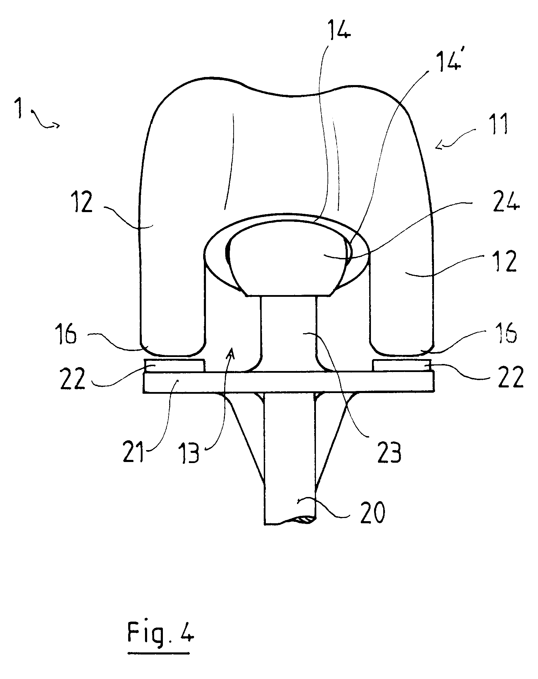

Si on se réfère également aux figures 3 et 4 on peut voir que l'implant tibial 2 comporte une tige intramédullaire 20 surmontée d'une embase tibiale 21 sur laquelle sont solidarisés de façon amovible, par des moyens connus en soi, deux demi-plateaux 22.

Centralement et postérieurement, entre les deux demi-plateaux 22, l'embase tibiale 21 comporte un ergot 23, non visible sur la figure 3, à l'extrémité duquel est fixée une tête sphérique 24 qui comporte à cet effet un orifice 25 qui peut être conique, la tête sphérique 24 étant d'un diamètre égal à celui de la cavité hémisphérique 14' de la cupule 14, ce diamètre pouvant varier de 10 à 40 mm.

Après implantation des implants fémoral 1 et tibial 2, ceux-ci sont assemblés par insertion de la tête sphérique 24 dans la cavité hémisphérique 14' de la cupule 14.

Lors de l'opération, des têtes sphériques 24 de profondeurs d'orifice 25 différentes sont à la disposition du chirurgien, de manière qu'il puisse choisir celle qui est la mieux adaptée a la tension ligamentaire. Le chirurgien dispose également de demi-plateaux 22 d'épaisseurs différentes permettant, après sélection de la tête sphérique 24, de choisir les demi-plateaux 22 dont l'épaisseur est la mieux adaptée à laisser subsister un jeu minimum entre les condyles 12 et les demi-plateaux 22.

Sur les figures 1 et 2 on peut voir que la cupule 14 comporte un bord 17 la prolongeant au-delà du diamètre de la cavité hémisphérique 14', ce bord 17 étant creusé de deux gorges 18 et 19 dont la largeur permet le passage de l'ergot 23, diamétralement opposées en regard de l'échancrure intercondylienne 13, de manière à ne pas limiter les mouvements en flexion et en extension.

Une telle prothèse permet que les contraintes soient appliquées uniquement au couple formé par la tête sphérique 24 et la cavité hémisphérique 14', interdisant tout jeu latéral, tandis que les mouvements dans le plan frontal sont limités au jeu existant entre les condyles 12 et les plateaux tibiaux 22, comme on peut le voir sur la figure 4.

Toutes les rotations dans le plan sagittal ainsi que les rotations internes ou externes du tibia par rapport au fémur sont autorisées, sous contraintes ou sans contraintes.

## Revendications

1. Prothèse de genou à articulation sphérique, notamment prothèse de remplacement d'une prothèse implantée antérieurement, comprenant d'une part un implant fémoral (1) présentant une partie condylienne (11) dans laquelle est formée une cavité centrale (15) ouverte en regard d'une échancrure intercondylienne (13), et dans laquelle est disposée une cupule (14) présentant une ouverture hémisphérique (14'), et d'autre part un implant tibial (2) comportant une embase tibiale (21) sur laquelle est solidarisé un plateau (22) en matière plastique, un ergot (23) faisant en outre saillie de l'embase tibiale entre les plateaux précités, caractérisée en ce que la cupule (14) contient une tête sphérique (24) dont le diamètre est égal à l'ouverture (14') de la cupule (14), et qui présente un orifice (25) permettant de coiffer l'ergot par ladite tête sphérique.

2. Prothèse selon la revendication 1 caractérisée en ce que la cupule (14) est prolongée par des bords (17) dans lesquels sont creusées deux gorges (18, 19), dont la largeur permet le passage de l'ergot (23), diamétralement opposées en regard de l'échancrure intercondylienne (13).

3. Prothèse selon la revendication 1 caractérisée en ce que la cupule (14) est à rétention de manière à retenir la tête sphérique (24).

4. Prothèse selon l'une quelconque des revendications précédentes caractérisée en ce que la cupule (14) est libre en mouvement dans la cavité (15).

5. Prothèse selon l'une quelconque des revendications précédentes caractérisée en ce que la fixation de la tête sphérique (24) sur l'ergot (23) est réalisée par emmanchement conique.

6. Prothèse selon l'une quelconque des revendications précédentes caractérisée en ce que le plateau (22) est mobile en rotation et en translation sur l'embase tibiale (21).

7. Prothèse selon l'une quelconque des revendications précédentes, caractérisée en ce que le plateau (22) est formé de deux demi-plateaux placés de part et d'autre de l'ergot central (23).

8. Prothèse selon l'une quelconque des revendications précédentes, caractérisée en ce que l'implant fémoral (1) comporte une partie trochléenne (13') prolongeant sa partie bicondylienne (11).

## Claims

1. A knee prosthesis with spherical articulation, particularly a replacement prosthesis for a previously implanted prosthesis, comprising on the one hand a femoral implant (1) having a condylar part (11) in which is formed a central cavity (15) which is open with respect to an intercondylar notch (13) and in which is arranged a cup (14) having a hemispherical opening (14'), and on the other hand a tibial implant (2) comprising a tibial seating (21) on which is fixed a plate (22) made plastic material, a lug (23) also forming a projection of the tibial seating between the aforementioned plates, characterised in that the cup (14) contains a spherical head (24) the diameter of which is equal to the opening (14') of the cup (14) and which has an orifice (25) allowing the lug to be covered by said spherical head.

2. A prosthesis according to claim 1, characterised in that the cup (14) is extended by edges (17) in which are hollowed out two grooves (18, 19), the width of which allows the passage of the lug (23), said grooves being diametrically opposed with respect to the intercondylar notch (13).

3. A prosthesis according to claim 1, characterised in that the cup (14) is a retaining cup such as to retain the spherical head (24).

4. A prosthesis according to any of the preceding claims, characterised in that the cup (14) is freely movable within the cavity (15).

5. A prosthesis according to any of the preceding claims, characterised in that the spherical head (24) is fixed to the lug (23) by conical coupling.

6. A prosthesis according to any of the preceding claims, characterised in that the plate (22) is capable of rotational and translational movement on the tibial seating (21).

7. A prosthesis according to any of the preceding claims, characterised in that the plate (22) is formed from two half-plates placed on either side of the central lug (23).

8. A prosthesis according to any of the preceding claims, characterised in that the femoral implant (1) comprises a trochlear part (13') extending its bicondylar part (11).

## Patentansprüche

1. Knieprothese mit Kugelgelenk, insbesondere Prothese zum Ersatz einer früher implantierten Prothese, bestehend einerseits aus einem Femoralimplantat (1) mit einem Kondylenteil (11), in dem ein zentraler Hohlraum (15) gebildet ist, der gegenüber einem Zwischenkondylenausschnitt (13) offen ist und in dem eine Kuppel (14) mit einer halbkugelförmigen Öffnung (14') angeordnet ist, und andererseits aus einem Tibialimplantat (2) mit einem Tibialaufsatz (21), auf dem ein Teller (22) aus Kunststoff befestigt ist, wobei außerdem auf dem Tibialaufsatz zwischen den Tellern ein Stift (23) hervorsteht, dadurch gekennzeichnet, daß die Kuppel (14) einen Kugelkopf (24) enthält, dessen Durchmesser gleich der Öffnung (14') der Kuppel (14) ist und der eine Öffnung (25) aufweist, die das Aufsetzen des Kugelkopfs auf den Stift gestattet.

2. Prothese nach Anspruch 1, dadurch gekennzeichnet, daß die Kuppel (14) durch Ränder (17) verlängert ist, in denen zwei Nuten (18, 19) vorgesehen sind, deren Breite den Durchgang des Stifts (23) gestattet und die gegenüber dem Zwischenkondylenausschnitt (13) einander diametral entgegengesetzt sind.

3. Prothese nach Anspruch 1, dadurch gekennzeichnet, daß die Kuppel (14) eine Zurückhaltungskuppel ist, so daß sie den Kugelkopf (24) zurückhält.

4. Prothese nach einem der vorhergehenden Ansprüche, dadurch gekennzeichnet, daß die Kuppel (14) in dem Hohlraum (15) frei beweglich ist.

5. Prothese nach einem der vorhergehenden Ansprüche, dadurch gekennzeichnet, daß die Befestigung des Kugelkopfs (24) auf dem Stift (23) durch Ineinanderstecken konischer Bereiche stattfindet.

6. Prothese nach einem der vorhergehenden Ansprüche, dadurch gekennzeichnet, daß der Teller (22) auf dem Tibialaufsatz (21) rotations- und translationsbeweglich ist.

7. Prothese nach einem der vorhergehenden Ansprüche, dadurch gekennzeichnet, daß der Teller (22) von zwei zu beiden Seiten des zentralen Stifts (23) angeordneten Tellerhälften gebildet wird.

8. Prothese nach einem der vorhergehenden Ansprüche, dadurch gekennzeichnet, daß das Femoralimplantat (1) einen Trochleateil (13') aufweist, der seinen Bikondylenteil (11) verlängert.
